**Europäisches Patentamt**

**European Patent Office**

**Office européen des brevets**

(19)

(11)  **EP 1 270 716 A1**

(12)  **EUROPÄISCHE PATENTANMELDUNG**

(43) Veröffentlichungstag:
  **02.01.2003  Patentblatt 2003/01**

(51) Int Cl.⁷: **C12M 1/34**, G01N 9/26

(21) Anmeldenummer: **02014428.3**

(22) Anmeldetag: **28.06.2002**

(84) Benannte Vertragsstaaten:
  **AT BE CH CY DE DK ES FI FR GB GR IE IT LI LU MC NL PT SE TR**
  Benannte Erstreckungsstaaten:
  **AL LT LV MK RO SI**

(30) Priorität: **29.06.2001  DE 10131158**

(71) Anmelder:
  • **Licht, Stephan, Dipl.-Ing.**
    **13597 Berlin (DE)**
  • **Licht, Horst, Dipl.-Ing.**
    **59320 Ennigerloh (DE)**

(72) Erfinder:
  • **Licht, Stephan, Dipl.-Ing.**
    **13597 Berlin (DE)**
  • **Licht, Horst, Dipl.-Ing.**
    **59320 Ennigerloh (DE)**

(74) Vertreter: **Cohausz & Florack**
  **Patentanwälte**
  **Kanzlerstrasse 8a**
  **40472 Düsseldorf (DE)**

(54)  **Verfahren zur kontinuierlichen Überwachung und Regelung von Gärprozessen**

(57)  Verfahren zur Bestimmung der Dichte von Gärflüssigkeiten (1a), umfassend folgende Schritte:

(a) Messen der Druckdifferenz, die zwischen mindestens zwei Messpunkten (5,6) unterschiedlicher Höhe in der Gärflüssigkeit (1a) besteht, über einen piezoresistiven Differenzdrucksensor (2) und

(b) Berechnen der Dichte aus Druckdifferenz und Höhenabstand (7) der Messpunkte

und Verfahren zur kontinuierlichen Überwachung und Regelung von Gärprozessen, welches folgende Schritte umfasst:

(a) Bestimmen der Dichte einer Gärflüssigkeit (1a) gemäß einem Verfahren nach einem der Ansprüche 1 bis 11 und/oder einer Vorrichtung nach einem der Ansprüche 12 bis 21;

(b) Bestimmen der Differenz von zu Beginn des Gärprozesses bestimmter Dichte und aktueller, im laufenden Gärprozess bestimmter Dichte.

(c) Bestimmen eines aktuellen Führungsparameters wie Zucker-, Alkohol- oder Säuregehalt der Gärflüssigkeit durch Korrelation der nach Schritt (b) ermittelten Dichtedifferenz zum Anfangswert des Führungsparameters, welcher zu Beginn des Gärungsprozesses bestimmt wurde

sowie Vorrichtungen zur Durchführung dieser Verfahren.

Fig.1

**Beschreibung**

Die Erfindung betrifft ein Verfahren zur Bestimmung der Dichte von Gärflüssigkeiten sowie ein Verfahren zur kontinuierlichen Überwachung und Regelung von Gärprozessen. Die Erfindung betrifft ferner Vorrichtungen zur Durchführung dieser Verfahren.

Bei der Herstellung von durch alkoholische Gärung erhaltenen Getränken wie Wein oder Bier wird im Fermentationsprozess Zucker in Alkohol umgewandelt. Aus Gründen der Qualitätssicherung ist es insbesondere bei der Weinherstellung erwünscht, dass der Fermentationsprozess so kontrolliert wie möglich abläuft, d.h. die Umwandlung von Zucker in Alkohol soll gleichmäßig über eine bestimmte Zeit zu einer vom Winzer vorgegebenen Zuckerabbaurate (g/Tag) erfolgen. Hierzu ist eine laufende Überwachung des Fermentationsprozesses anhand des Zucker- und/oder Alkoholgehaltes erforderlich, um gegebenenfalls die Fermentationsbedingungen wie beispielsweise die Mosttemperatur anzupassen.

Insbesondere in kleineren Weinkellereien erfolgt die Überwachung des Fermentationsprozesses in aller Regel manuell.

Dies geschieht in aller Regel dadurch, dass der Winzer in regelmäßigen Abständen mit Hilfe eines Thermometers die Temperatur und mit Hilfe einer Oechsle-Waage den ungefähren Zuckergehalt des Mostes misst. Eine Oechsle-Waage ist ein Aräometer, welches das spezifische Gewicht von Trauben- oder Obstsäfte für eine bestimmte Temperatur, beispielsweise 15°C, angibt. Anhand des gemessenen spezifischen Mostgewichtes lässt sich der ungefähre Zuckergehalt des Mostes ermitteln. Dieser Wert ist jedoch nicht genau und eignet sich lediglich für eine grobe Kontrolle des Fermentationsprozesses.

Aus der US 5 204 262 ist ein Verfahren zur computergesteuerten Fermentationskontrolle bekannt, bei dem der Gärungsverlauf durch kontinuierliche Messung des Alkoholgehaltes über einen diffusionsgesteuerten Ethanolsensor bestimmt wird. Nachteilig an dem beschriebenen Verfahren ist der hohe technische Aufwand und die damit verbundenen Kosten. Nachteilig ist ferner, dass Rückschlüsse auf den biologischen Säureabbau nicht möglich sind.

Ferner sind Versuche unternommen worden, über kontinuierliche Dichtemessungen eine Kontrolle des Fermentationsverlaufes zu erzielen. So ist beispielsweise eine von der Firma Liquosystems in Deutschland vertriebene und in deren Firmenprospekt "Dichtesonde Stand 2001" näher beschriebene Dichtesonde bekannt, die in den Gärtank eingehängt wird und eine elektronische Messung des Absolutdruckes an drei verschiedenen Messpunkten im Gärtank vornimmt. Aus den einzelnen Druckmessungen wird kontinuierlich die Dichte des Mostes bestimmt. Nachteilig an dieser Dichtesonde ist, dass Füllstandsänderungen und Änderungen der Eintauchtiefe der Dichtesonde im Gärtank zu Änderungen der Absolutdrücke führt, die um das Vielfache größer sind, als die zu messenden Druckdifferenzen. Die mit Hilfe der Sonde gemessenen Dichtewerte sind daher anfällig für Störfaktoren und ermöglichen daher keinen genauen Rückschluss auf den exakten Zuckergehalt und Fermentationsverlauf.

Aufgabe der Erfindung ist es, ein exaktes, einfaches und preiswertes Verfahren zur Bestimmung der Dichte einer Gärflüssigkeit durch kontinuierliche Dichtemessung zu schaffen, welches die vorgenannten, aus dem Stand der Technik bekannten Nachteile nicht aufweist. Insbesondere soll sich der ermittelte Dichtewert zur exakten und kontinuierlichen Berechnung von Führungsgrößen wie Zucker- und Alkoholgehalt während des Gärverfahrens eignen und somit eine automatische Überwachung und Regelung des Gärprozesses ermöglichen.

Erfindungsgemäß wird diese Aufgabe durch ein Verfahren gelöst, das folgende Schritte umfasst:

(a) Messen der Druckdifferenz, die zwischen mindestens zwei Messpunkten unterschiedlicher Höhe in der Gärflüssigkeit besteht, über einen piezoresistiven Differenzdrucksensor und

(b) Berechnen der Dichte aus Druckdifferenz und Höhenabstand zwischen den beiden Messpunkten.

Der im erfindungsgemäßen Verfahren eingesetzte piezoresistive Differenzdrucksensor eignet sich überraschend gut zur Messung von Dichten in Gärflüssigkeiten. Im Gegensatz zu aus dem Stand der Technik bekannten Dichtesonden, die nach dem Absolutdruckmessverfahren arbeiten, ermöglicht der erfindungsgemäß eingesetzte Differenzdrucksensor eine exakte, von Störgrößen und Umgebungsveränderungen im wesentlichen unabhängige Dichtemessung. Die Dichtemessung ist so exakt, dass sie - wie nachfolgend näher beschrieben werden wird - als Messgröße zur kontinuierlichen Überwachung des Gärungsprozesses herangezogen werden kann, welche direkte Rückschlüsse auf den Zucker-, Alkohol- und Säuregehalt der Gärflüssigkeit erlaubt. Das erfindungsgemäße Verfahren erfordert ferner keinen grossen apparativen Aufwand und ist mit verhältnismässig preiswerten Mitteln durchführbar. Es ist damit auch geeignet, in kleineren Weinkellereien eingesetzt zu werden, die in der Regel aus Kostengründen von aufwendigen Fermentationskontrollanlagen absehen.

Das Verfahren kann zur Dichtemessung in beliebigen Fermentationsprozessen, beispielsweise bei der Herstellung von Weinen und Bieren, eingesetzt werden. Insbesondere kann das Verfahren sowohl in kontinuierlichen als auch in im Batch-Verfahren durchgeführten Prozessen eingesetzt werden. Vorzugsweise wird die erfindungsgemäße Dichtebestimmung in einem für chargenweise Gärungsprozesse vorgesehenen Behälter durchgeführt.

In Schritt (a) des Dichtemessverfahrens wird zunächst die Druckdifferenz, die die zwischen mindestens zwei Messpunkten unterschiedlicher Höhe in der Gärflüssigkeit besteht, gemessen. Die Druckdifferenzmessung erfolgt mit Hilfe eines piezoresistiven Differenzdrucksensors. Derartige Sensoren sind dem Fachmann allgemein bekannt und beispielsweise in den US 4 411 158, US 3 764 950, US 3 820 401, US 3 930 412 und US 3 918 019 beschrieben. Vorzugsweise handelt es sich bei dem piezoresistiven Sensor um einen von zwei Seiten mit Druck beaufschlagten Sensor. Es können beliebige Drucksensoren eingesetzt werden, die nach dem piezoresistiven Prinzip arbeiten, ohne auf die vorgenannten, im Stand der Technik beschriebenen Sensoren beschränkt zu sein.

Die Differenzdruckmessung kann beispielsweise derart erfolgen, dass der Differenzdrucksensor mit Verbindungselementen wie Rohren oder Schläuchen verbunden ist, die zu dem jeweiligen Messpunkt in der Gärflüssigkeit führen. Die Verbindungselemente können beispielsweise mit einem Übertragungsmedium wie einer Flüssigkeit oder einem Gas gefüllt sein, welches den in der Gärflüssigkeit an dem jeweiligen Messpunkt vorherrschenden Druck auf den Sensor Messbar überträgt. Vorzugsweise sind die Verbindungselemente mit einem Gas, insbesondere mit Luft, gefüllt.

Um eine gleichbleibende Messgenauigkeit zu gewährleisten wird vorzugsweise während des Verfahrens Sorge dafür getragen, dass die Verbindungselemente stets zu einem gleichbleibenden Stand mit dem Übertragungsmedium gefüllt sind. Vorzugsweise sind die Verbindungselemente vollständig, d.h. bis zum äußeren Ende, mit dem Übertragungsmedium gefüllt.

Bei Verwendung von Flüssigkeiten als Übertragungsmedium kann zwischen dem Übertragungsmedium und der Gärflüssigkeit eine Trennmembran vorgesehen werden, um eine Durchmischung der beiden Flüssigkeiten zu verhindern.

Es hat sich als besonders vorteilhaft herausgestellt, die Verbindungselemente mit einem Gas, beispielsweise mit Luft oder $CO_2$, zu füllen und die in die Gärflüssigkeit ragenden Enden der Verbindungselemente offen und ohne Anordnung einer Membran auszugestalten, so dass eine unmittelbare Übertragung des in der Flüssigkeit vorherrschenden Druckes auf das gasförmige Übertragungsmedium erfolgen kann. Durch Verzicht auf die Membran wird eine erhöhte Meßgenauigkeit erreicht. Vorzugsweise sind die Verbindungselemente derart im Gärtank angeordnet, dass sie mit ihren Enden im Wesentlichen vertikal nach unten weisen, so dass das in den Verbindungselemente enthaltene gasförmige Übertragungsmedium nicht über den mit Gärflüssigkeit befüllten Gärtank entweichen kann. Dies kann etwa dadurch erreicht werden, dass die Verbindungselemente derart angeordnet werden, dass sie mit ihren offenen Enden vertikal nach unten abgeschrägt von außen in das Innere des Gärtanks hineinragen. Es ist vorteilhaft, die in die Gärflüssigkeit ragenden Enden der Verbindungselemente offen und ohne jegliche Membran auszugestalten, damit äußere Einflüsse auf das gasförmige Übertragungsmedium ausgeglichen werden können. Dies ist deswegen erforderlich, weil sich das Übertragungsmedium bei einer Temperaturerhöhung der Gärflüssigkeit oder Umgebungsluft ausdehnt. Das überschüssige gasförmige Übertragungsmedium kann so über die offenen Enden der Verbindungselemente in den Gärtank entweichen. Bei einer Absenkung der Temperatur zieht sich das gasförmige Übertragungsmedium zusammen, was dazu führt, dass die Verbindungselemente nicht mehr vollständig mit dem Übertragungsmedium gefüllt sind. Das fehlende Übertragungsmedium wird entweder automatisch durch im Gärtank aufsteigendes Gärgas ersetzt, wobei die in das Verbindungselement eingedrungene Gärflüssigkeit verdrängt wird oder kann extern durch Zuführen von Übertragungsmedium ersetzt werden. Letzteres kann entweder manuell, beispielsweise durch einen Blasebalg oder eine Handpumpe, erfolgen oder automatisch durch zwei Magnetventile, die an ein Gasnetz angeschlossen sind. Bei angesteuertem Ventil wird Übertragungsmedium durch die Verbindungselemente in den Gärtank gedrückt, wodurch das Verbindungselement wieder vollständig mit gasförmigem Übertragungsmedium gefüllt wird. Bei abgefallenem Ventil ist das Verbindungselement mit dem piezoresistiven Drucksensor verbunden, so dass der Differenzdruck gemessen werden kann. Das externe Füllen der Verbindungselemente mit gasförmigem Übertragungsmedium kann auch zum Freiblasen benutzt werden, was insbesondere dann vorteilhaft ist, wenn die Gefahr besteht, dass sich die Verbindungselemente zusetzen (z. B. durch Schalen oder Kerne bei der Maischegärung). Beim manuellen Zuführen von gasförmigem Übertragungsmedium können anstelle von Magnetventilen auch 3-Wege-Hähne oder T-Stücke und Handventile eingesetzt werden.

Die mit dem Drucksensor verbundenen Verbindungselemente können beispielsweise über einen Stutzen oder einen Blindkegelflansch von außen in den Gärtank eingebracht werden. Alternativ können die Verbindungselemente auch über den Gärspund in den Gärtank eingebracht werden.

Gemäß einer bevorzugten Ausführungsform der Erfindung beträgt das Verhältnis von Höhenunterschied zwischen den Messpunkten (in cm) zu Messbereichsspanne des Differenzdrucksensors (in mbar) 0,75 bis 1,0, insbesondere 0,8 bis 0,9, beträgt. Hierdurch wird eine optimale Messgenauigkeit speziell für die Zwecke der Dichtebestimmung von Gärflüssigkeiten erzielt.

So kann der Höhenunterschied zwischen den Messpunkten bei der Differenzdruckmessung etwa 60 cm betragen und/oder der Differenzdrucksensor einen Messbereich von 0-70 mbar aufweisen.

Gemäß einer weiteren Ausführungsform der Erfindung kann die Messgenauigkeit des erfindungsgemäßen Verfahrens weiterhin dadurch erhöht werden, indem dem Differenzdrucksensor ein Differenzverstärker nachgeschaltet ist, der den Messbereich des Differenzdrucksensors von einer Untergrenze bis zu einer Obergrenze verstärkt. Derartige Differenzverstärker sind dem Fachmann allgemein bekannt und beispielsweise in der US 4 174 639 beschrieben. Es können beliebige Differenzverstärker eingesetzt werden, die dazu geeignet sind, den Messbereich des Differenzdrucksensors in einem gewünschten Bereich zu verstärken, ohne auf die im vorgenannten Stand der Technik beschriebenen Differenzverstärker beschränkt zu sein.

Vorzugsweise wird der Differenzverstärker so gewählt, dass der von diesem zu verstärkende Messbereich eine Untergrenze (in mbar) aufweist, die etwa dem Produkt aus dem Höhenunterschied zwischen den Messpunkten (in cm) und einem Faktor von 0,80 bis 1,10, insbesondere von 0,90 bis 1,00, entspricht.

Vorzugsweise wird der Differenzverstärker so gewählt, dass der von diesem zu verstärkende Messbereich eine Untergrenze (in mbar) aufweist, die etwa dem Produkt aus dem Höhenunterschied zwischen den Messpunkten (in cm) und einem Faktor von 1,00 bis 1,30, insbesondere von 1,10 bis 1,20, entspricht.

Durch die Wahl der vorgenannten Grenzen für die Differenzverstärkung wird eine optimale Messgenauigkeit speziell für die Zwecke der Dichtebestimmung von Gärflüssigkeiten erzielt.

Anhand der gemessenen Druckdifferenz $\Delta p$, der Erdbeschleunigung g und der vorzugsweise konstant gehaltenen Höhendifferenz $\Delta h$ der Messpunkte kann anhand folgender Formel die Dichte d der Gärflüssigkeit berechnet werden:

$$d = \frac{\Delta p}{\Delta h \cdot g}$$

Gemäß einer bevorzugten Ausführungsform der Erfindung wird ferner die Temperatur der Gärflüssigkeit gemessen. Die Messung kann beispielsweise über einen Thermosensor erfolgen. Anhand der gemessenen Temperatur kann die Dichte auf beliebige Temperaturen umgerechnet werden. Vorzugsweise wird die Dichte normiert auf 20°C berechnet.

Die Vorrichtung zur Druckdifferenzmessung und/oder der Thermosensor können entweder fest im Gärtank installiert oder auch lösbar, insbesondere als Handmessgeräte, ausgebildet sein. In letzteren Fall muss gewährleistet sein, dass die Messung bei stets gleichem Höhenabstand der Messpunkte erfolgt. Zweckmäßig wird dies durch starre Rohre erreicht, die von dem Differenzdrucksensor wegführen und deren Enden in unterschiedlicher Höhe nach unten in die Flüssigkeit ragen. Um genaue Messwerte zu gewährleisten, muss das Handmessgerät ferner so im Gärbehälter befestigt oder gehalten werden, dass sich der Höhenabstand der beiden Messpunkte während der Messung nicht ändert.

Es versteht sich, dass die Berechnung der Dichte mit Hilfe eines Mikroprozessors, insbesondere eines Mikrocomputers, vorgenommen werden kann und/oder auf einem Anzeigelement wie einem LCD-Display oder einem Monitor angezeigt werden können.

Vorteilhaft ist ferner vorgesehen, dass die gemessenen und/oder berechneten Werte auf einem Speichermedium in einer Datenbank gespeichert werden.

Gegenstand der Erfindung ist ferner eine Vorrichtung zur Durchführung eines Verfahrens zur Bestimmung der Dichte von Gärflüssigkeiten, umfassend:

(a) einen Gärbehälter zur Aufnahme von Gärflüssigkeit und

(b) einen piezoresistiven Differenzdrucksensor zur Messung der in der Gärflüssigkeit bestehenden Druckdifferenz zwischen zwei in unterschiedlicher Höhe im Gärbehälter befindlichen Messpunkten.

Vorzugsweise umfasst die Vorrichtung ferner einen in dem Gärbehälter angeordnetem Thermosensor, welcher zur Messung der Temperatur der Gärflüssigkeit dient.

Wie zuvor beschrieben sind dabei zur Übertragung der Drücke von den jeweiligen Messpunkten zur Membran des piezoresistiven Differenzdrucksensors vorzugsweise einem Übertragungsmedium wie Gas oder Flüssigkeit gefüllte Verbindungsmittel wie Rohre oder Schläuche vorgesehen, die in einem bestimmten Höhenabstand voneinander derart im Gärbehälter angeordnet sind, dass sie mit der Gärflüssigkeit in Kontakt kommen.

Wie zuvor beschrieben beträgt das Verhältnis von Höhenunterschied zwischen den Messpunkten (in cm) zu Messbereichsspanne des Differenzdrucksensors (in mbar) dabei vorzugsweise 0,75 bis 1,00, insbesondere 0,80 bis 0,90.

Wie zuvor beschrieben ist dem Differenzdrucksensor ferner vorzugsweise ein Differenzverstärker nachgeschaltet, der den Messbereich des Differenzdrucksensors von einer Untergrenze bis zu einer Obergrenze verstärkt.

Wie zuvor beschrieben beträgt die Untergrenze (in mbar) der Messbereichsverstärkung vorzugsweise etwa dem Produkt aus dem Höhenunterschied zwischen den Messpunkten (in cm) und einem Faktor von 0,80 bis 1,10, insbesondere von 0,90 bis 1,00, entspricht.

Wie zuvor beschrieben beträgt die Obergrenze (in mbar) der Messbereichsverstärkung vorzugsweise etwa dem Produkt aus dem Höhenunterschied zwischen den Messpunkten (in cm) und einem Faktor von 1,00 bis 1,30, insbesondere von 1,10 bis 1,20, entspricht.

Die Vorrichtung ist vorzugsweise mit einem Mikroprozessor ausgestattet, der mit mindestens dem piezoresistiven Differenzdrucksensor und/oder dem Thermosensor verbunden ist. Darüber hinaus kann sie auch ein Speichermedium umfassen, das mit mindestens dem Mikroprozessor verbunden ist und die gemessenen und/oder berechneten Werte in einer Datenbank speichert.

Schließlich kann die Vorrichtung ein Anzeigeelement wie ein LCD-Display oder einen Monitor umfassen, das mit dem Differenzdrucksensor, dem Thermosensor, dem Speichermedium und/oder dem Mikroprozessor verbunden ist und die gemessenen, berechneten und/oder gespeicherten Werte anzeigt.

Die Erfindung umfasst ferner ein Verfahren zur kontinuierlichen Überwachung und Regelung von Gärprozessen, welches folgende Schritte umfasst:

(a) Bestimmen der Dichte einer Gärflüssigkeit durch kontinuierliche Differenzdruckmessung während des Gärprozesses wie zuvor beschrieben;

(b) Bestimmen der Differenz von zu Beginn des Gärprozesses bestimmter Dichte und aktueller, im laufenden Gärprozess bestimmter Dichte.

(c) Bestimmen eines aktuellen Führungsparameters wie Zucker- oder Alkoholgehalt der Gärflüssigkeit durch Korrelation der nach Schritt (b) ermittelten Dichtedifferenz zum Anfangswert des Führungsparameters, welcher zu Beginn des Gärungsprozesses bestimmt wurde.

Das erfindungsgemässe Verfahren sich dadurch aus, dass es anhand der über Differenzdruckmessung ermittelten Dichte der Gärflüssigkeit eine gezielte Überwachung und Regelung des Gärungsprozesses erlaubt.

Bei der Durchführung des erfindungsgemässen Verfahrens kann beispielsweise so vorgegangen werden, dass bei Beginn des Gärungsprozesses zunächst einmal die Dichte der Gärflüssigkeit über die vorgenannte Differenzdruckmessung bestimmt wird. Zu Beginn des Gärungsprozesses wird ferner mindestens eine Führungsgröße der Gärflüssigkeit wie Zucker-, Alkohol-, Nichtzucker- oder Säuregehalt analytisch oder auf sonstige Weise bestimmt. Diese Anfangswerte werden festgehalten und dienen als Korrelationsgröße für die Berechnung der Führungsgrößen zu einem späteren Zeitpunkt im Gärungsprozess.

Sodann wird aus der zu Beginn des Gärungsprozesses ermittelten Dichte und der zu einem beliebigen späteren Zeitpunkt im Gärungsprozess ermittelten Dichte eine Dichtedifferenz gebildet. Vorzugsweise wird diese Differenz kontinuierlich in gleichen Zeitabständen während des gesamten Gärprozesses bestimmt. Durch Korrelation der Dichtedifferenz mit dem zu Beginn des Gärungsprozesses ermittelten Wert der Führungsgröße lässt sich der Wert der Führungsgröße zu dem späteren Zeitpunkt bestimmen und so kontinuierlich über den gesamten Gärungsprozess verfolgen. Vorzugsweise wird eine kontinuierliche Überwachung anhand des Zuckergehaltes als Führungsgröße durchgeführt.

Genaue Werte für die Führungsgrößen können erzielt werden, wenn die Bestimmung der Dichte in Schritt (a), der Dichtedifferenz in Schritt (b) und/oder des Führungsparameters in Schritt (c) unter Berücksichtigung von für den jeweiligen Gärungsprozess spezifischen Korrektorfaktoren berechnet wird.

Die Berücksichtigung der Korrekturfaktoren in den einzelnen Schritten (a), (b) und/oder (c) trägt dem Umstand Rechnung, dass keine lineare, sondern eine dynamische, von einer Vielzahl von Parametern im Gärungsprozess beeinflusste Abhängigkeit zwischen ermittelter Dichte und dem tatsächlichen Wert der Führungsgröße wie Zuckergehalt besteht. Um exakte Werte für die Führungsgrößen zu erhalten ist es daher empfehlenswert, die jeweils berechneten Werte mit Hilfe von Korrekturfaktoren zu bereinigen. Die eingesetzten Korrekturfaktoren sind für den jeweiligen Gärungsprozess spezifisch und schliessen unter anderem folgende Faktoren ein:

- Hefegehalt und -aktivität,
- Prozesstemperatur,
- Feststoffgehalt,
- Gehalt an gelöster und ungelöster Kohlensäure,
- Alkoholzunahme während des Gärprozesses,
- Gehalt an gelöster und ungelöster Kohlensäure,
- Säureabbau,
- Nichtzuckeranteile.

Die Berücksichtigung der Korrekturfaktoren kann statisch oder dynamisch erfolgen. Im Fall von statischen Korrekturfaktoren bleibt der Wert des Korrekturfaktors während des gesamten Gärungsprozesses gleich. Im Fall von dynamischen Korrekturfaktoren wird der Wert des Korrekturfaktors dem Gärungsverlauf angepasst und ändert sich gegebenenfalls im Verlauf des Gärungsprozesses.-Die genauesten Werte erzielt man durch Einbeziehung von dynamischen Korrekturfaktoren.

Es versteht sich, dass das Verfahren vorzugsweise unter Einbeziehung eines Mikrocomputers durchgeführt wird, der die einzelnen Werte, Führungsgrößen und/oder Korrekturfaktoren erfasst, berechnet, auf einem Speichermedium speichert, auswertet, überwacht und/oder auf einem Anzeigeelement anzeigt.

Der Mikrocomputer hat dabei vorzugsweise Zugriff eine oder mehrere Datenbanken, in denen die zur Berechnung erforderlichen Daten gespeichert sind.

In einer ersten Datenbank können beispielsweise die zu Beginn des Gärungsprozesses experimentell bestimmten Anfangsdaten der Gärflüssigkeit wie Zucker-, Alkohol- und Säuregehalt, Hefemenge, Hefeart, Füllmenge, Nichtzuckeranteile etc. und/oder Angaben zum beabsichtigten Gärungsverlauf wie beabsichtigte Zuckerabbaurate oder bestimmte Temperaturvorgaben gespeichert sein.

In einer zweiten Datenbank können beispielsweise die für bestimmte Einsatzstoffe und Prozessbedingungen spezifischen Korrekturfaktoren gespeichert sein.

In einer dritten Datenbank können beispielsweise die gemessenen und berechneten Werte wie Dichte, Temperatur, Zucker-, Alkohol- und Säuregehalt gespeichert sein.

Stellt der Computer Abweichungen von für den Gärungsverlauf vorgegebenen Werten fest, wird der Benutzer gemäß einer weiteren Ausgestaltung des Verfahrens über eine Warnvorrichtung, z. B. über ein akustisches Signal, darüber informiert und kann dann manuell in den Gärungsprozess, z. B. durch Änderung der Mosttemperatur, eingreifen und diesen regeln.

Vorzugsweise erfolgt diese Regelung des Gärprozesses jedoch automatisch und wird vom Computer automatisch bei Abweichung von den vorgegebenen Gärungsparametern und/oder in Abhängigkeit von den in den Schritten (a), (b) und/oder (c) ermittelten Werten vorgesehen.

Die Regelung des Gärprozesses kann beispielsweise durch Veränderung der Gärtemperatur erfolgen. Zweckmäßigerweise kann dies durch Abkühlen der Gärflüssigkeit über eine Kühlplatte oder einen Kühlmantel, eine Berieselungsvorrichtung oder durch Erwärmen der Gärflüssigkeit über einen Heizstab oder eine Wärmetauscherplatte erfolgen.

Eine weitere Möglichkeit der Regelung des Gärprozesses besteht in kontrolliertem Einperlen von Gasen in die Gärflüssigkeit (Flotation).

Gegenstand der Erfindung ist ferner eine Vorrichtung zur Durchführung des zuvor beschriebenen Verfahrens zur kontinuierlichen Überwachung und Regelung von Gärprozessen, umfassend mindestens:

(a) einen piezoresistiven Differenzdrucksensor zur Messung der in einer Gärflüssigkeit bestehenden Druckdifferenz zwischen zwei in unterschiedlicher Höhe in einem Gärbehälter befindlichen Messpunkten;

(b) einen Thermosensor;

(c) einen Mikrocomputer, der mit dem Differenzdrucksensor und dem Thermosensor verbunden ist und Zugriff hat auf ein Speichermedium umfassend

- eine erste Datenbank, in der die zu Beginn des Gärungsprozesses experimentell bestimmten Anfangsdaten der Gärflüssigkeit wie Zucker-, Alkohol- und Säuregehalt, Hefemenge, Hefeart, Füllmenge, Nichtzuckeranteile und/oder Angaben zum beabsichtigten Gärungsverlauf wie beabsichtigte Zukerabbaurate gespeichert sind, und/oder

- eine zweite Datenbank, in der für bestimmte Einsatzstoffe und Prozessbedingungen spezifische Korrekturfaktoren gespeichert sind, und/oder

- eine dritte Datenbank, in der die gemessenen und berechneten Werte gespeichert sind,

wobei der Mikrocomputer mit einem Algorithmus ausgestattet ist, welcher anhand der gemessenen und gespeicherten Daten die Berechnung und Auswertung von gärungsprozessspezifischen Führungsparametern erlaubt.

Die Erfindung wird im folgenden anhand eines Ausführungsbeispiels näher erläutert, wobei auf die beigefügten Zeichnungen Bezug genommen wird.

In den Zeichnungen zeigen

Fig. 1     eine schematische Darstellung einer Vorrichtung zur Durchführung des erfindungsgemäßen Verfahrens und

Fig. 2     eine perspektivische Darstellung eines Ausschnitts der in Fig. 1 dargestellten Vorrichtung, in der zusätzlich eine Vorrichtung zum automatischen Befüllen der Meßrohre mit gasförmigem Übertragungsmedium vorgesehen ist.

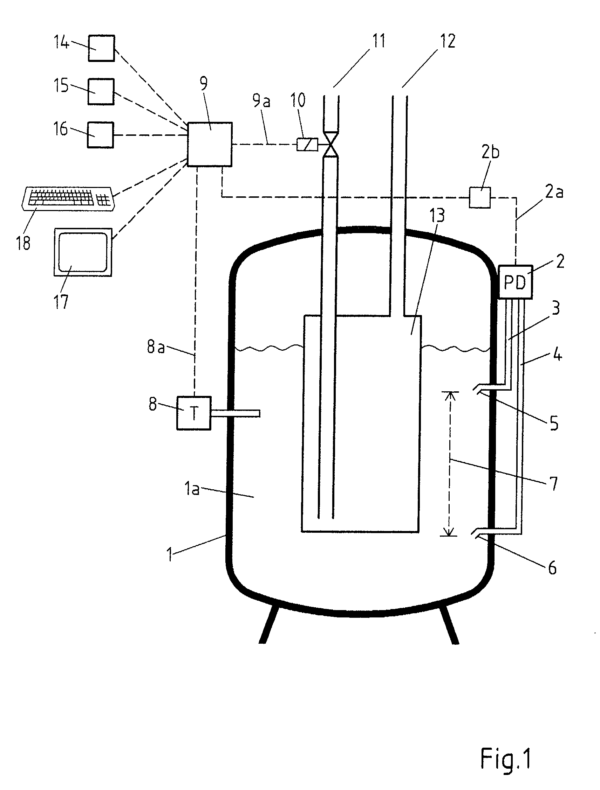

Fig. 1 zeigt eine zur Durchführung des erfindungsgemäßen Verfahrens zur kontinuierlichen Überwachung und Regelung von Gärprozessen geeignete Vorrichtung mit einem Gärbehälter 1, einem piezoresistiven Differenzdrucksensor 2 und einem Thermosensor 8. Der Differenzdrucksensor 2 misst die Druckdifferenz, die zwischen zwei in einem bestimmten Höhenabstand 7 voneinander angeordneten Messpunkten 5,6 in der Gärflüssigkeit 1a besteht. Zur Übertragung des jeweiligen Drucks auf den Differenzdrucksensor 2 ist dieser über starre Rohre 3,4, die in den Gärbehälter führen und nach unten abgewinkelte Enden aufweisen, welche die Messpunkte 5,6 darstellen, verbunden, wobei die Rohre 3,4 zur Übertragung des Drucks vollständig mit Luft gefüllt sind. Das von dem Differenzdrucksensor bei der Druckmessung erzeugte elektrische Signal wird über eine elektrische Leitung 2a an einen Mikrocomputer 9 übertragen, der das Signal erfasst und weiterverarbeitet.

Der Gärtank 1 ist ferner mit einem Thermosensor 8 ausgestattet, der von außen in den Gärtank 1 führt und die Temperatur der Gärflüssigkeit 1a misst. Das von dem Thermosensor 8 erzeugte elektrische Signal wird über eine elektrische Leitung 8a an den Mikrocomputer 9 übertragen, der das Signal erfasst und weiterverarbeitet.

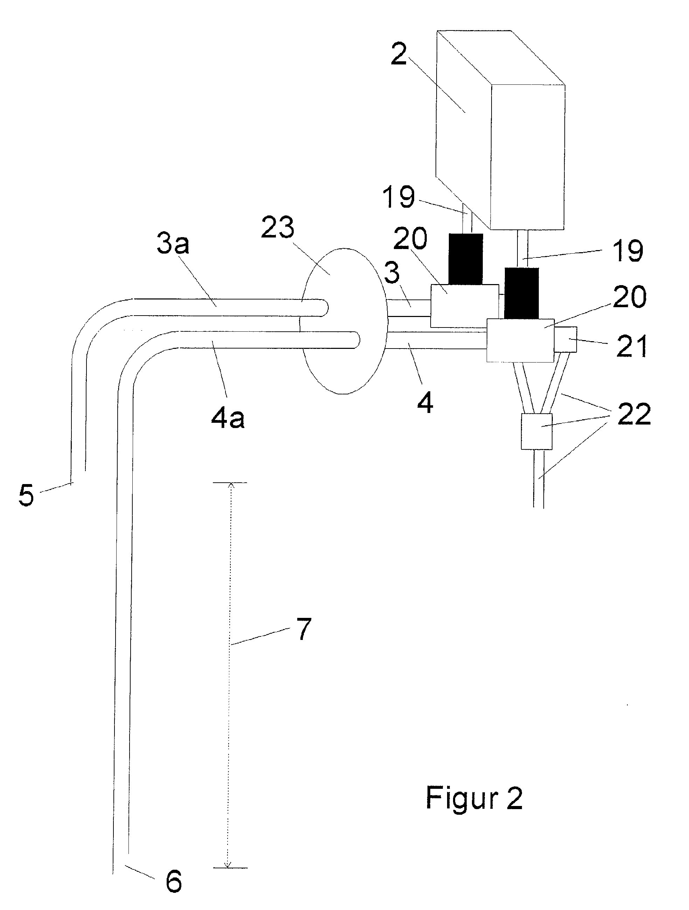

Der Mikrocomputer 9 hat Zugriff auf verschiedene Datenbanken 14,15,16, in denen verschiedene zuvor beispielhaft erläuterte Daten gespeichert sind, die für die Berechnung und Überwachung des Gärungsverlaufes wichtig sind. Anhand der gemessenen und der in den Datenbanken 14,15,16 gespeicherten Daten errechnet der Mikrocomputer 9 bestimmte Führungsgrößen wie beispielsweise den aktuellen Zuckergehalt der Gärflüssigkeit 1a und vergleicht den berechneten Wert mit einem vorgegebenen Sollwert für den Zuckerabbau. Der Sollwert wird entweder vom Benutzer des Verfahrens fest vorgegeben oder vom Mikrocomputer anhand der in den Datenbanken 14,15,16 vorhandenen Daten dynamisch entsprechend dem Gärungsverlauf berechnet. Entspricht der berechnete Wert nicht dem Sollwert, so leitet der Mikrocomputer 9 Schritte ein, die den Gärungsprozess regeln. In der abgebildeten Ausführungsform erfolgt die Regelung dadurch, dass der Mikrocomputer 9 über eine elektrische Leitung 9a elektrische Signale an ein Regelungsventil 10 sendet und dieses entweder öffnet oder schließt. Das Regelungsventil 10 regelt die Kalt- und/oder Warmwasserzufuhr 11 für eine im Inneren des Gärbehälters 1 vorgesehene Wärmetauscherplatte 13, die die Gärflüssigkeit 1a bei zu schneller Fermentation kühlt und damit den Gärungsprozess verlangsamt bzw. bei zu langsamer Fermentation heizt und damit den Gärprozess beschleunigt. Das Kühlwasser fließt über ein Rohr 12 wieder aus der Kühlplatte 13 ab. **[0059]** In Fig. 2 ist eine im erfindungsgemäßen Verfahren einsetzbare Anlage zum automatischen Belüften der in den Gärtank ragenden Meßrohre 3,4 dargestellt, wie sie insbesondere bei der Maischegärung eingesetzt werden kann. Die Meßrohre 3,4 ragen über einen Blindkegelflansch 23 über einen horizontalen

Abschnitt 3a,4a und einen vertikalen Abschnitt in den Gärbehälter 1. Die in den Gärbehälter 1 ragenden Rohre 3a,4a sind vertikal nach unten gebogen und weisen an ihren die Meßpunkte 5,6 darstellenden Enden eine abgeschrägte Öffnung auf. Die Abschrägung der Enden ist deshalb von Vorteil, da überschüssige Luft nicht als Blase am Ende der Meßrohre hängen bleiben kann. Der Meßfehler wird dadurch geringer. Die außerhalb des Gärbehälters 1 befindlichen Rohre 3,4 sind über ein 3/2-Wege-Magnetventil 20 über ein Verbindungsrohr 19 einerseits mit dem Drucksensor 2 und andererseits mit der Luftleitung 21 verbunden, welche über eine Luftdrossel 22 zur Reduzierung und Dosierung des Drucks mit einer nicht abgebildeten Druckluftflasche verbunden ist. Anstelle eines Luftnetzes kann auch ein $CO_2$-Netz eingesetzt werden. Bei angesteuertem Magnetventil 20 wird Luft durch die Rohre 3,4 bzw. 3a,4a in den Gärbehälter 1 gedrückt. Bei abgefallenem Magnetventil 20 sind die Rohre 3,4 über die Leitungen 19 mit dem Drucksensor 2 verbunden, so dass der Differenzdruck gemessen werden kann. Die in Fig. 2 beschriebene Belüftungsanlage ist insbesondere bei der Maischegärung vorteilhaft, bei der die Gefahr besteht, dass sich die Öffnungen an den Meßpunkten 5,6 durch Schalen oder Kerne zusetzen. Durch die Belüftungsanlage können die zugesetzten Öffnungen 5,6 in einfacher Weise freigeblasen werden.

**Patentansprüche**

1. Verfahren zur Bestimmung der Dichte von Gärflüssigkeiten (1a), umfassend folgende Schritte:

    (a) Messen der Druckdifferenz, die zwischen mindestens zwei Messpunkten (5,6) unterschiedlicher Höhe in der Gärflüssigkeit (1a) besteht, über einen piezoresistiven Differenzdrucksensor (2) und

    (b) Berechnen der Dichte aus Druckdifferenz und Höhenabstand (7) der Messpunkte.

2. Verfahren nach Anspruch 1, **dadurch gekennzeichnet, dass** als weiterer Schritt die Temperatur der Gärflüssigkeit (1a) gemessen wird und die Dichte auf eine bestimmte Temperatur, insbesondere auf 20°C, normiert berechnet wird.

3. Verfahren nach einem der vorhergehenden Ansprüche, **dadurch gekennzeichnet, dass** die Dichtebestimmung in einem für die chargenweise Gärung vorgesehenen Behälter (1) erfolgt.

4. Verfahren nach einem der vorhergehenden Ansprüche, **dadurch gekennzeichnet, dass** in Schritt (a) die Druckdifferenz zwischen den Messpunkten (5,6) über mit Gas oder Flüssigkeit gefüllte Rohre oder Schläuche (3,4) gemessen wird, über welche die in der Gärflüssigkeit (1a) bestehenden Drücke von den jeweiligen Messpunkten (5,6) zur Membran des piezoresistiven Differenzdrucksensors (2) übertragen werden.

5. Verfahren nach einem der vorhergehenden Ansprüche, **dadurch gekennzeichnet, dass** in Schritt (a) das Verhältnis von Höhenunterschied (7) zwischen den Messpunkten (in cm) zu Messbereichsspanne des Differenzdrucksensors (2) (in mbar) 0,75 bis 1,00, insbesondere 0,80 bis 0,90, beträgt.

6. Verfahren nach einem der vorhergehenden Ansprüche, **dadurch gekennzeichnet, dass** in Schritt (a) dem Differenzdrucksensor (2) ein Differenzverstärker (2b) nachgeschaltet ist, der den Messbereich des Differenzdrucksensors (2) von einer Untergrenze bis zu einer Obergrenze verstärkt.

7. Verfahren nach Anspruch 6, **dadurch gekennzeichnet, dass** die Untergrenze (in mbar) etwa dem Produkt aus dem Höhenunterschied (7) zwischen den Messpunkten (5,6)(in cm) und einem Faktor von 0,80 bis 1,10, insbesondere von 0,90 bis 1,00, entspricht.

8. Verfahren nach Anspruch 6 oder 7, **dadurch gekennzeichnet, dass** die Obergrenze (in mbar) etwa dem Produkt aus dem Höhenunterschied (7) zwischen den Messpunkten (5,6) (in cm) und einem Faktor von 1,00 bis 1,30, insbesondere von 1,10 bis 1,20, entspricht.

9. Verfahren nach einem der vorhergehenden Ansprüche, **dadurch gekennzeichnet, dass** die Berechnung der Dichte mittels eines Mikroprozessors (9) erfolgt.

10. Verfahren nach einem der vorhergehenden Ansprüche, **dadurch gekennzeichnet, dass** die gemessenen und/oder berechneten Werte auf einem Speichermedium in einer Datenbank (14,15,16) gespeichert werden.

11. Verfahren nach einem der vorhergehenden Ansprüche, **dadurch gekennzeichnet, dass** die gemessenen und/oder berechneten Werte auf einem Anzeigeelement (17), beispielsweise einem LCD-Display oder Monitor, angezeigt werden.

12. Vorrichtung zur Durchführung eines Verfahrens gemäß einem der Ansprüche 1 bis 11, umfassend mindestens

    (a) einen Gärbehälter (1) zur Aufnahme von Gärflüssigkeit und

    (b) einen piezoresistiven Differenzdrucksensor (2) zur Messung der in der Gärflüssigkeit (1a) bestehenden Druckdifferenz zwischen zwei in unterschiedlicher Höhe im Gärbehälter (1) befindlichen Messpunkten (5,6);

13. Vorrichtung nach Anspruch 12, **dadurch gekennzeichnet, dass** ein in dem Gärbehälter (1) angeordneter Thermosensor (8) zur Messung der Temperatur der Gärflüssigkeit (1a) vorgesehen ist.

14. Vorrichtung nach Anspruch 12 oder 13, **dadurch gekennzeichnet, dass** zur Übertragung der Drücke von den jeweiligen Messpunkten (5,6) zur Membran des piezoresistiven Differenzdrucksensors (2) mit Gas oder Flüssigkeit gefüllte Rohre und/oder Schläuche (3,4) vorgesehen sind, die in einem bestimmten Höhenabstand (7) voneinander derart im Gärbehälter (1) angeordnet sind, dass sie mit der Gärflüssigkeit (1a) in Kontakt kommen.

15. Vorrichtung nach einem der Ansprüche 12 bis 14, **dadurch gekennzeichnet, dass** das Verhältnis von Höhenunterschied (7) zwischen den Messpunkten (5,6) (in cm) zu Messbereichsspanne des Differenzdrucksensors (2) (in mbar) 0,75 bis 1,00, insbesondere 0,80 bis 0,90, ist.

16. Vorrichtung nach einem der Ansprüche 12 bis 15, **dadurch gekennzeichnet, dass** dem Differenzdrucksensor (2) ein Differenzverstärker (2b) nachgeschaltet ist, der den Messbereich des Differenzdrucksensors (2) von einer Untergrenze bis zu einer Obergrenze verstärkt.

17. Vorrichtung nach Anspruch 16, **dadurch gekennzeichnet, dass** die Untergrenze (in mbar) etwa dem Produkt aus dem Höhenunterschied (7) zwischen den Messpunkten (5,6) (in cm) und einem Faktor von 0,80 bis 1,10, insbesondere von 0,90 bis 1,00, entspricht.

18. Vorrichtung nach Anspruch 16 oder 17, **dadurch gekennzeichnet, dass** die Obergrenze (in mbar) etwa dem Produkt aus dem Höhenunterschied (7) zwischen den Messpunkten (5,6) (in cm) und einem Faktor von 1,00 bis 1,30, insbesondere von 1,10 bis 1,20, entspricht.

19. Vorrichtung nach einem der Ansprüche 12 bis 18, **dadurch gekennzeichnet, dass** sie einen Mikroprozessor (9) umfasst, der mit mindestens dem piezoresistiven Differenzdrucksensor (2) und/oder dem Thermosensor (8) verbunden ist.

20. Vorrichtung nach Anspruch 19, **dadurch gekennzeichnet, dass** sie ein Speichermedium umfasst, das mit mindestens dem Mikroprozessor verbunden ist und die gemessenen und/oder berechneten Werte in einer Datenbank (14,15,16) speichert.

21. Vorrichtung nach einem der Ansprüche 12 bis 20, **dadurch gekennzeichnet, dass** sie ein Anzeigeelement (17) umfasst, das mit dem Differenzdrucksensor (2), dem Thermosensor (8), dem Speichermedium und/oder dem Mikroprozessor (9) verbunden ist und die gemessenen, berechneten und/oder gespeicherten Werte anzeigt.

22. Verfahren zur kontinuierlichen Überwachung und Regelung von Gärprozessen, welches folgende Schritte umfasst:

    (a) Bestimmen der Dichte einer Gärflüssigkeit (1a) gemäß einem Verfahren nach einem der Ansprüche 1 bis 11 und/oder einer Vorrichtung nach einem der Ansprüche 12 bis 21;

    (b) Bestimmen der Differenz von zu Beginn des Gärprozesses bestimmter Dichte und aktueller, im laufenden Gärprozess bestimmter Dichte.

    (c) Bestimmen eines aktuellen Führungsparameters wie Zucker-, Alkohol- oder Säuregehalt der Gärflüssigkeit durch Korrelation der nach Schritt (b) ermittelten Dichtedifferenz zum Anfangswert des Führungsparameters, welcher zu Beginn des Gärungsprozesses bestimmt wurde.

23. Verfahren nach Anspruch 22, **dadurch gekennzeichnet, dass** die Bestimmung der Dichtedifferenz in Schritt (b) und/oder des Führungsparameters in Schritt (c) unter Berücksichtigung eines Korrekturfaktors für mindestens eines der folgenden Parameter erfolgt:

- Hefegehalt und -aktivität,
- Prozesstemperatur,
- Feststoffgehalt,
- Gehalt an gelöster und ungelöster Kohlensäure,
- Alkoholzunahme während des Gärprozesses,
- Gehalt an gelöster und ungelöster Kohlensäure,
- Säureabbau,
- Nichtzuckeranteile.

24. Verfahren nach Anspruch 22 oder 23, **dadurch gekennzeichnet, dass** ein Mikrocomputer (9) vorgesehen ist, der die einzelnen Werte, Führungsgrößen und/oder Korrekturfaktoren erfasst, berechnet, auf einem Speichermedium speichert, auswertet, überwacht und/oder auf einem Anzeigeelement (17) anzeigt.

25. Verfahren nach Anspruch 24, **dadurch gekennzeichnet, dass** der Mikrocomputer Zugriff auf eine erste Datenbank (14) hat, in der die zu Beginn des Gärungsprozesses experimentell bestimmten Anfangsdaten der Gärflüssigkeit wie Zucker-, Alkohol- und Säuregehalt, Hefemenge, Hefeart, Nichtzuckeranteile, Füllmenge etc. und/oder Angaben zum beabsichtigten Gärungsverlauf wie beabsichtigte Zuckerabbaurate gespeichert sind.

26. Verfahren nach einem der Ansprüche 24 oder 25, **dadurch gekennzeichnet, dass** der Mikrocomputer Zugriff auf eine zweite Datenbank (15) hat, in der für bestimmte Einsatzstoffe und Prozessbedingungen spezifische Korrekturfaktoren gespeichert sind.

27. Verfahren nach einem der Ansprüche 24 bis 26, **dadurch gekennzeichnet, dass** der Mikrocomputer Zugriff auf eine dritte Datenbank (16) hat, in der die gemessenen und berechneten Werte gespeichert sind.

28. Verfahren nach einem der Ansprüche 24 bis 27, **dadurch gekennzeichnet, dass** als weiterer Schritt eine automatische Regelung des Gärprozesses in Abhängigkeit von den in den Schritten (a) bis (c) ermittelten Werten erfolgt.

29. Verfahren nach Anspruch 28, **dadurch gekennzeichnet, dass** die Regelung des Gärprozesses durch Veränderung der Gärtemperatur erfolgt.

30. Verfahren nach Anspruch 29, **dadurch gekennzeichnet, dass** die Veränderung der Gärtemperatur durch Abkühlen der Gärflüssigkeit über eine Kühlplatte (13) oder einen Kühlmantel, Berieselung des Gärbehälters mit Wasser, oder durch Erwärmen der Gärflüssigkeit über einen Heizstab oder eine Wärmetauscherplatte erfolgt.

31. Verfahren nach Anspruch 28, **dadurch gekennzeichnet, dass** die Regelung des Gärprozesses durch kontrolliertes Einperlen von Gasen in die Gärflüssigkeit (Flotation) erfolgt.

32. Vorrichtung zur Durchführung eines Verfahrens gemäß einem der Ansprüche 22 bis 31, umfassend mindestens:

(a) einen piezoresistiven Differenzdrucksensor (2) zur Messung der in einer Gärflüssigkeit (1a) bestehenden Druckdifferenz zwischen zwei in unterschiedlicher Höhe in einem Gärbehälter (1) befindlichen Messpunkten (5,6);

(b) einen Thermosensor (8) zur Messung der Temperatur der Gärflüssigkeit (1a) und

(c) einen Mikrocomputer (9), der mit dem Differenzdrucksensor (2) und dem Thermosensor (8) verbunden ist und Zugriff hat auf ein Speichermedium umfassend

- eine erste Datenbank (14), in der die zu Beginn des Gärungsprozesses experimentell bestimmten Anfangsdaten der Gärflüssigkeit wie Zucker-, Alkohol- und Säuregehalt, Hefemenge, Hefeart, Füllmenge und/oder Angaben zum beabsichtigten Gärungsverlauf wie beabsichtigte Zuckerabbaurate gespeichert sind, und/oder

- eine zweite Datenbank (15), in der für bestimmte Einsatzstoffe und Prozessbedingungen spezifische Korrekturfaktoren gespeichert sind, und/oder

- eine dritte Datenbank (16), in der die gemessenen und berechneten Werte gespeichert sind,

wobei der Mikrocomputer (9) mit einem Algorithmus ausgestattet ist, welcher anhand der gemessenen und gespeicherten Daten die Berechnung und Auswertung von gärungsprozessspezifischen Führungsparametern erlaubt.

**33.** Vorrichtung nach Anspruch 32, **dadurch gekennzeichnet, dass** der Differenzdrucksensor (2) die in einem der Ansprüche 14 bis 18 genannten Merkmale aufweist.

**34.** Vorrichtung nach Anspruch 32 oder 33, **dadurch gekennzeichnet, dass** der Mikrocomputer (9) mit einem Eingabemittel (18) und einem Anzeigeelement (17) verbunden ist.

**35.** Vorrichtung nach einem der Ansprüche 32 bis 34, **dadurch gekennzeichnet, dass** eine Warnvorrichtung vorgesehen ist, welche bei Abweichung der Führungsparameter vom vorgegebenen Gärungsverlauf akustische oder optische Warnsignale abgibt.

**36.** Vorrichtung nach einem der Ansprüche 32 bis 35, **dadurch gekennzeichnet, dass** mindestens Regelungselement vorgesehen ist, welches bei Abweichung der Führungsparameter vom vorgegebenen Gärungsverlauf Einfluss auf die Prozessbedingungen wie Gärtemperatur nimmt.

**37.** Vorrichtung nach Anspruch 36, **dadurch gekennzeichnet, dass** das Regelungselement eine in die Gärflüssigkeit tauchende Kühl- oder Heizplatte (13) oder ein außen am Gärbehälter vorgesehener Kühl- oder Heizmantel oder eine Berieselungsvorrichtung ist.

Fig.1

Figur 2

Europäisches
Patentamt

## EUROPÄISCHER RECHERCHENBERICHT

Nummer der Anmeldung

EP 02 01 4428

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | FR 2 641 865 A (CHAMPAGNE STATION OENOTECHNIQU) 20. Juli 1990 (1990-07-20) * das ganze Dokument * | 1-37 | C12M1/34 G01N9/26 |
| X | FR 2 639 710 A (AVENTEL SARL ;CADEAC GILLES (FR)) 1. Juni 1990 (1990-06-01) * das ganze Dokument * | 1-37 | |
| P,X | DE 201 10 719 U (LICHT HORST ;LICHT STEPHAN (DE)) 27. September 2001 (2001-09-27) * das ganze Dokument * | 1-37 | |

**RECHERCHIERTE SACHGEBIETE (Int.Cl.7)**

C12M
G01N

Der vorliegende Recherchenbericht wurde für alle Patentansprüche erstellt

| Recherchenort | Abschlußdatum der Recherche | Prüfer |
|---|---|---|
| DEN HAAG | 24. September 2002 | Coucke, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 02 01 4428

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-09-2002

| Im Recherchenbericht angeführtes Patentdokument | | Datum der Veröffentlichung | Mitglied(er) der Patentfamilie | | Datum der Veröffentlichung |
|---|---|---|---|---|---|
| FR 2641865 | A | 20-07-1990 | FR | 2641865 A1 | 20-07-1990 |
| FR 2639710 | A | 01-06-1990 | FR | 2639710 A1 | 01-06-1990 |
| DE 20110719 | U | 27-09-2001 | DE | 20110719 U1 | 27-09-2001 |

EPO FORM P0461

Für nähere Einzelheiten zu diesem Anhang : siehe Amtsblatt des Europäischen Patentamts, Nr.12/82